# EUROPEAN PATENT APPLICATION

(11) **EP 3 747 365 A1**
(43) Date of publication of application: **09.12.2020**
(21) Application number: 19747165.9
(22) Date of filing: 28.01.2019
(51) Int. Cl.: A61B 5/16, A61B 5/026

(54) **ELECTRONIC APPARATUS, ESTIMATION SYSTEM, CONTROL METHOD, AND CONTROL PROGRAM**

(30) Priority: 30.01.2018 JP 2018013958
(71) Applicant: Kyocera Corporation, Fushimi-ku, Kyoto-shi, Kyoto 612-8501 (JP)
(72) Inventor: MITSUI Katsuhiro, Kyoto-shi, Kyoto 612-8501 (JP)
(74) Representative: Viering, Jentschura & Partner mbB Patent- und Rechtsanwälte
(86) International application number: PCT/JP2019/002797
(87) International publication number: WO 2019/151195

(57) **Abstract**

An electronic apparatus includes a measurement unit configured to measure a blood flow volume of a subject and a controller configured to estimate a sleep state of the subject based on the blood flow volume of the subject.

## Description

### CROSS REFERENCE TO RELATED APPLICATION

This application claims priority to and the benefit of Japanese Patent Application No. 2018-013958 filed on January 30, 2018, the entire contents of which are incorporated herein by reference.

### TECHNICAL FIELD

The present disclosure relates to an electronic apparatus, an estimation system, a control method, and a control program.

### BACKGROUND

Conventionally, devices for detecting a sleep state are known. For example, Patent Document 1 discloses an apparatus that detects a sleep state by measuring a brainwave. However, an electroencephalograph that measures a brainwave is not always easy to use.

### CITATION LIST

### Patent Literature

PTL 1: JP-A-2013-121489

### SUMMARY

An electronic apparatus according to an embodiment includes a measurement unit configured to measure blood flow information of a subject, and a controller configured to estimate a sleep state of the subject based on a variance of the blood flow information.

An estimation system according to an embodiment includes a blood flow information measurement apparatus equipped with a measurement unit configured to measure blood flow information of a subject, and an estimation apparatus equipped with a controller configured to estimate a sleep state of the subject based on a variance of the blood flow information.

A control method according to an embodiment includes measuring blood flow information of a subject, and estimating a sleep state of the subject, based on a variance of the blood flow information.

A control program according to an embodiment causes a computer to measure blood flow information of a subject and estimate a sleep state of the subject, based on a variance of the blood flow information.

### BRIEF DESCRIPTION OF THE DRAWINGS

FIG. 1 is a schematic diagram illustrating an example external appearance of an electronic apparatus according to a first embodiment;
FIG. 2 is a cross-sectional view taken along line A-A of a measurement apparatus illustrated in FIG. 1;
FIG. 3 is a functional block diagram illustrating an example schematic configuration of the electronic apparatus illustrated in FIG. 1;
FIG. 4 is a graph schematically illustrating an example brainwave related to sleep;
FIG. 5A is a schematic diagram illustrating a method for determining a range of measurement data associated with calculation of a variance;
FIG. 5B is a schematic diagram illustrating a method for determining a range of the measurement data associated with calculation of the variance;
FIG. 6 is a schematic diagram illustrating a method for determining a range of measurement data associated with calculation of a plurality of variances;
FIG. 7 is a schematic diagram illustrating a sleep state estimation method;
FIG. 8 is a schematic diagram illustrating a method for determining a range of data associated with calculation of a representative value of a variance;
FIG. 9A is a graph illustrating a result of experiment indicating a relationship between blood flow information and a sleep state;
FIG. 9B is a graph illustrating a result of experiment indicating a relationship between the blood flow information and the sleep state; and
FIG. 10 is a flowchart illustrating an example of a sleep state estimation operation executed by a controller illustrated in FIG. 3.

### DETAILED DESCRIPTION

An electronic apparatus, an estimation system, a control method, and a control program according to the present disclosure described below can improve convenience.

Hereinafter, a detailed description will be given with reference to the drawings. In the present specification, components that have the same function on the left and right sides and collectively described without being distinguished from each other will be denoted by reference numerals omitting an alphabet a or b. For example, when a first measurement unit 112a and a second measurement unit 112b are not distinguished from each other, they will be collectively referred to as a measurement unit 112.

### First Embodiment

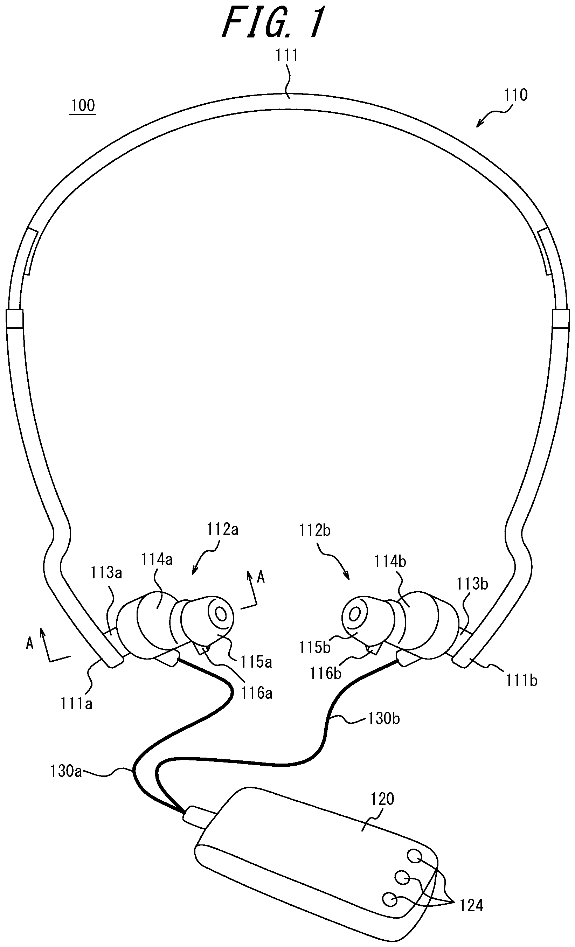

Hereinafter, a first embodiment of an electronic apparatus 100 will be described with reference to FIG. 1 to FIG. 7.

FIG. 1 is a schematic diagram illustrating an example external appearance of the electronic apparatus 100 according to the first embodiment. The electronic apparatus 100 includes a measurement apparatus (a blood flow information measurement apparatus) 110 and an estimation apparatus 120. The measurement apparatus 110 and the estimation apparatus 120 may be communicatively connected to each other via cables 130a and 130b. The measurement apparatus 110 and the estimation apparatus 120 can transmit and receive various signals and electric power via the cables 130a and 130b. The measurement apparatus 110 and the estimation apparatus 120 do not necessarily need to be connected to each other via the cables 130a and 130b. The measurement apparatus 110 and the estimation apparatus 120 may be communicatively connected to each other via, for example, wireless networking or a combination of wired and wireless networking.

The measurement apparatus 110 includes a wearing unit 111, a first measurement unit 112a, and a second measurement unit 112b. The measurement apparatus 110 measures information about blood flow of a subject (hereinafter, also referred to as "blood flow information") using the first measurement unit 112a and the second measurement unit 112b in a state of being worn on the subject. The blood flow information may be any one of, for example, a blood flow volume, a pulsating blood flow wave height, a stroke volume, and a blood volume. The pulsating blood flow wave height is a difference between a maximum value and a minimum value of a blood flow volume in one heartbeat. The stroke volume is, for example, a blood flow volume per heartbeat. A unit of the blood flow information may be expressed by, for example, a volume per unit time or simply by a volume.

The measurement apparatus 110 can be worn on, for example, the head of the subject. In the present specification, a mode in which the measurement apparatus 110 is used in a state of being worn on the head of the subject will be described.

The wearing unit 111 has a mechanism for maintaining a wearing state of the measurement apparatus 110 on the subject. In the present embodiment, the wearing unit 111 has, for example, an arch shape as illustrated in FIG. 1. The subject can wear the measurement apparatus 110 and maintain the wearing state in such a manner that the wearing unit 111 grips the head of the subject. The wearing unit 111 may have a mechanism that allows adjustment of its length to the size of, for example, the head of the subject. The wearing unit 111 may be made of, for example, plastics or the like.

The first measurement unit 112a is connected to a first end portion 111a of the wearing unit 111 via a first connecting portion 113a, and the second measurement unit 112b is connected to a second end portion 111b of the wearing unit 111 via a second connecting portion 113b. That is, the wearing unit 111, the first measurement unit 112a, the second measurement unit 112b, the first connecting portion 113a, and the second connecting portion 113b are connected together as a whole and constitute the measurement apparatus 110 of one earphone type. The first connecting portion 113a is formed at a portion connecting the first measurement unit 112a and the first end portion 111a of the wearing unit 111. The second connecting portion 113b is formed at a portion connecting the second measurement unit 112b and the second end portion 111b of the wearing unit 111.

The first measurement unit 112a and the second measurement unit 112b include respective sensors configured to measure the blood flow information of the subject. Details of the sensors will be described later. The first measurement unit 112a and the second measurement unit 112b have the same functions. In the present specification, when the first measurement unit 112a and the second measurement unit 112b are not distinguished from each other, they will be collectively referred to as a measurement unit 112.

In the present embodiment, the measurement unit 112 measures the blood flow information in the ear of the subject. The measurement unit 112 may measure the blood flow information in, for example, the concha, the ear canal, the earlobe, or the tragus of the subject.

The first measurement unit 112a measures the blood flow information in, for example, the concha of the right ear of the subject. The first measurement unit 112a includes a main body 114a, an earpiece 115a, and a sensor 116a. The earpiece 115a is inserted into the external auditory meatus of the right ear of the subject in the wearing state of the measurement apparatus 110. The earpiece 115a is made of, for example, silicon. The sensor 116a is located on a portion of the main body 114a to come into contact with the concha of the right ear, which is serving as a test site, in a state in which the earpiece 115a is inserted into the external auditory meatus of the right ear. The main body 114a also functions as a casing for protecting a measurement mechanism (details of which will be described later) of the sensor 116a. The main body 114a may be made of, for example, plastics. The earpiece 115a maintains a contact state of the sensor 116a in contact with the concha of the ear serving as the test site, in a state of being inserted into the external auditory meatus of the right ear. The sensor 116a functions as a measurement unit configured to measure the blood flow information of the subject.

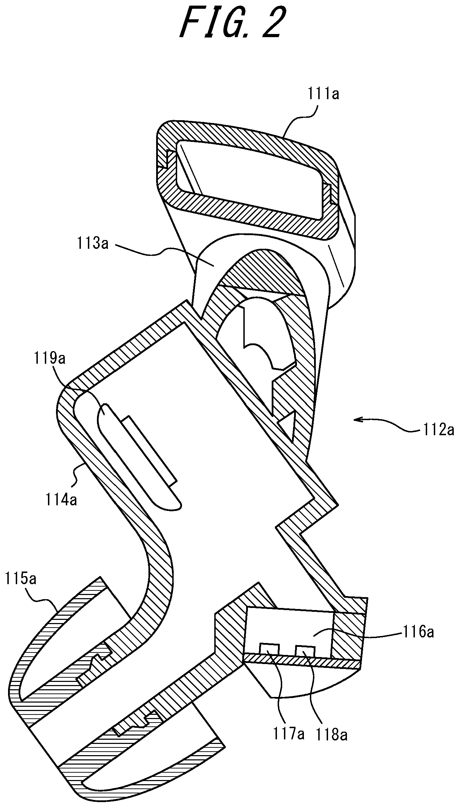

FIG. 2 is a cross-sectional view taken from line A-A of the measurement apparatus 110 illustrated in FIG. 1. That is, FIG. 2 is a partial cross-sectional view that includes the first measurement unit 112a configured to measure the blood flow information in the right ear. Note that some details of an internal mechanism of the first measurement unit 112a are omitted in FIG. 2.

As schematically illustrated in FIG. 2, the sensor 116a includes an optical emitter 117a and an optical detector 118a that constitute a measuring mechanism. The optical emitter 117a emits measurement light to the concha of the right ear serving as the test site. The optical detector 118a receives reflected light (scattered light) of measurement light from the tissues inside the concha. A photoelectric conversion signal of scattered light received by the optical detector 118a is transmitted to the controller 122 that is included in the estimation apparatus 120. The controller 122 may be included in the measurement apparatus 110. Details of the controller 122 will be described later.

The optical emitter 117a emits, for example, laser light. The optical emitter 117a irradiates the test site with, for example, laser light in a wavelength capable of detecting predetermined components contained in blood as measurement light. The optical emitter 117a is configured to be, for example, one LD (laser diode).

The optical detector 118a receives scattered light of measurement light from the test site as information (hereinafter referred to as biological information) obtained as a result of measurement conducted by the measurement apparatus 110 on the living body of the subject. The optical detector 118a is configured to be, for example, a PD (Photodiode).

As illustrated in FIG. 2, the main body 114a encloses a speaker 119a. The speaker 119a generates a sound based on an input sound signal. The sound signal is input from, for example, the estimation apparatus 120. The speaker 119a functions as a notification interface capable of notifying the subject of various information. The speaker 119a also functions as, in addition to as the notification interface, an output interface when the electronic apparatus 100 is connected to an external input device such as a music player to reproduce music.

The first connecting portion 113a connects the wearing unit 111 and the first measurement unit 112a at the first end portion 111a of the wearing unit 111. The first connecting portion 113a may include a member capable of attenuating vibration. Such a member capable of attenuating vibration includes, for example, a piece of spring, rubber, silicone resin, gel, cloth, sponge, paper, other members, or any combination thereof. The member capable of attenuating vibration may be, for example, a fluid-filled damper containing a fluid (i.e., liquid or gas). The fluid may be, for example, a viscous liquid. In a case in which the first connecting portion 113a includes a member capable of attenuating vibration, vibration transmitted to the first measurement unit 112a from the wearing unit 111 is attenuated. This suppresses a change in a contact state between the first measurement unit 112a and the test site and thus improves the measurement accuracy of the biological information.

The second measurement unit 112b measures the information regarding blood flow at, for example, the concha of the left ear of the subject. The second measurement unit 112b has a symmetrical structure with respect to, and the same function as, the first measurement unit 1112a. That is, the second measurement unit 112b includes a main body 114b, an earpiece 115b, a sensor 116b, and a speaker 119b. The main body 114b, the earpiece 115b, the sensor 116b, and the speaker 119b realize the respective functions of the main body 114a, the earpiece 115a, the sensor 116a, and the speaker 119a in the left ear. The sensor 116b functions as a measurement unit configured to measure the blood flow information of the subject. The sensor 116b includes an optical emitter 117b and an optical detector 118b. The functions of the optical emitter 117b and the optical detector 118b may be similar to the respective functions of the optical emitter 117a and the optical detector 118a. The second measurement unit 112b is connected to the second end portion 111b of the wearing unit 111 via the second connecting portion 113b. The second measurement unit 112b may have the same configuration as the first measurement unit 112a.

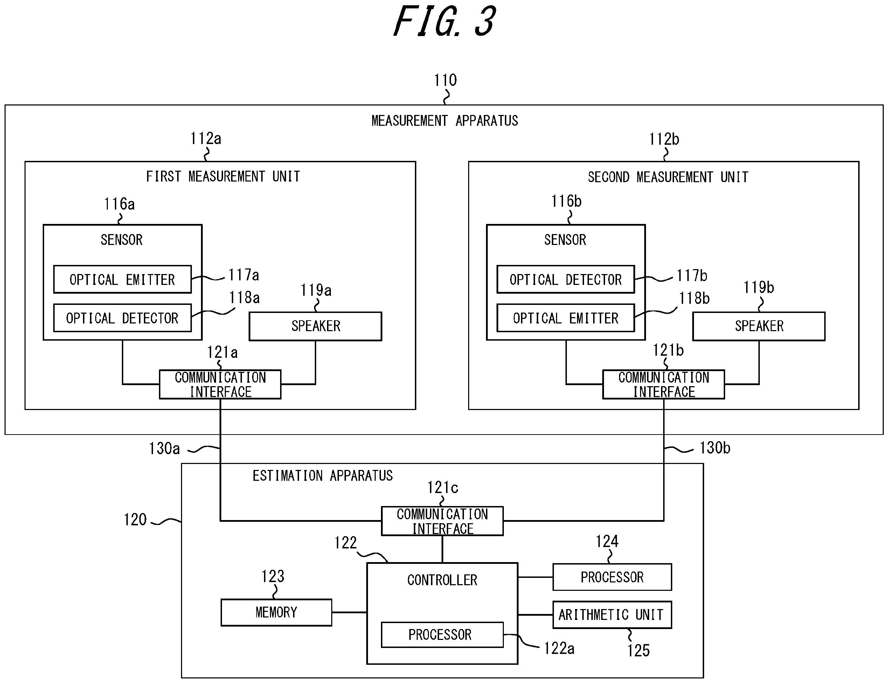

FIG. 3 is a functional block diagram illustrating an example schematic configuration of the electronic apparatus 100 illustrated in FIG. 1. Hereinafter, transmission and reception of various signals will be described with reference to FIG. 3.

The first measurement unit 112a includes a communication interface 121a. The communication interface 121a transmits and receives various signals by communicating with the estimation apparatus 120. In the present embodiment, the communication interface 121a communicates with the estimation apparatus 120 via the cable 130a. However, the communication interface 121a may communicate with the estimation apparatus 120 via, for example, wireless networking or a combination of wired and wireless networking. The communication interface 121a transmits, for example, a photoelectric conversion signal of scattered light received by the optical detector 118a to the estimation apparatus 120. The communication interface 121a receives, for example, a signal for causing the sensor 116a to carry out a biological information measurement operation from the estimation apparatus 120. The communication interface 121a receives, for example, a signal associated with a sound to be output from the speaker 119a from the estimation apparatus 120.

The second measurement unit 112b includes a communication interface 121b. In the present embodiment, the communication interface 121b communicates with the estimation apparatus 120 via the cable 130b. The communication interface 121b may have the same function as the communication interface 121a included in the first measurement unit 112a.

The estimation apparatus 120 includes a communication interface 121c, a controller 122, an arithmetic unit 125, a memory 123, and an input interface 124.

The communication interface 121c transmits and receives various signals by communicating with the measurement apparatus 110. In the present embodiment, the communication interface 121c communicates with the first measurement unit 112a and the second measurement unit 112b of the measurement apparatus 110 via the cables 130a and 130b, respectively. The communication interface 121c receives, for example, the photoelectric conversion signal of scattered light received by the optical detector 118 from the measurement apparatus 110. The communication interface 121c transmits, for example, a signal for causing the sensor 116 to carry out the biological information measurement operation to the measurement apparatus 110. The communication interface 121c transmits, for example, a signal associated with a sound to be output from the speakers 119 to the measurement apparatus 110.

The controller 122 includes at least one processor 122a configured to control and manage the electronic apparatus 100 in its entirety including each functional block thereof. The controller 122 includes at least one processor 122a configured to be a CPU (Central Processing Unit) or the like that executes a program defining a control procedure and realizes its function. Such a program is stored in, for example, the memory 123 or an external storage medium connected to the electronic apparatus 100.

The at least one processor 122a included in the controller 122 may be, for example, one IC (Integrated Circuit), or a plurality of ICs and/or discrete circuits (Discrete Circuits) that are communicatively connected. In such an embodiment, the processor 122a included in the controller 122 as one or more circuits or units may be configured to execute one or more data calculation procedures or operations in accordance with an instruction stored in a related memory.

In another example, the processor 122a may be firmware (e.g., a discrete logic component). The firmware may be configured to execute one or more data calculation procedures or operations.

In the above example, the processor 122a can operate according to various known techniques. The processor 122a may include one or more processors, controllers, microprocessors, microcontrollers, ASICs (Application-specific integrated circuits), digital signal processors, programmable logic devices, field programmable gate arrays, any combination of these devices or configurations thereof, or any combination of other known devices or configurations thereof, and may perform a function of the controller 122 described below.

The controller 122 calculates the blood flow information in the test site of the subject, based on the photoelectric conversion signal of scattered light received from the measurement apparatus 110. The controller 122 measures the blood flow information using, for example, the Doppler shift. Here, an example of blood flow information measurement techniques using the Doppler shift will be described below.

Light scattered by blood cells moving in tissues of a living body undergoes a frequency shift (the Doppler shift) that is proportional to a moving speed of the blood cells in the blood, based on the Doppler effect. The controller 122 detects a beat signal caused by light interference between scattered light from stationary tissues of the living body and scattered light from the blood cells moving in the tissues of the living body. The beat signal is expressed by a function of scattered light intensity and time. The controller 122 performs FFT (Fast Fourier transform) on the beat signal to generate a frequency spectrum (a power spectrum) with weighted frequency components. In the power spectrum of the beat signal, the number of high frequency components increases in proportion to a speed of the blood cell, and the power corresponds to an amount of the blood cells. Further, the controller 122 calculates a first moment by integrating the power spectrum of the beat signal with the frequency components and then performs normalizing by dividing the first moment by a DC signal component, thereby obtains the blood flow information. A calculation method according to the present embodiment is not limited to the above.

The controller 122 estimates a sleep state of the subject, based on calculated blood flow information. Details of a sleep state estimation operation executed by the controller 122 will be described later.

The memory 123 can be configured to be a semiconductor memory, a magnetic memory, or the like. The memory 123 stores various information and/or programs for operating the electronic apparatus 100. The memory 123 may also function as a working memory.

The input interface 124 is configured to receive an input operation from the subject and includes, for example, operation buttons (operation keys) as illustrated in FIG. 1. The input interface 124 may be configured to be, for example, a touch screen and may display an input area for receiving an input operation from the subject in a part of a display device and receive a touch input operation from the subj ect.

Incidentally, the controller 122 estimates the sleep state of the subject, as described above. Here, the sleep state of the subject will be described in detail.

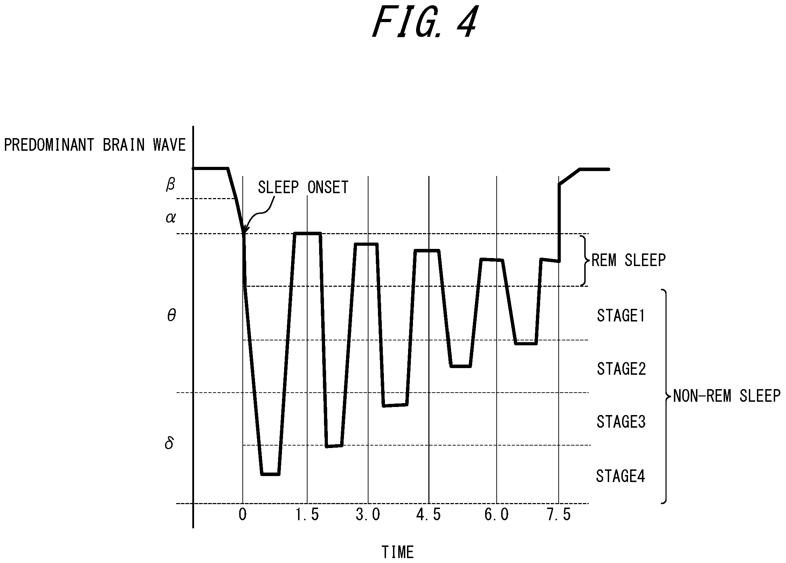

In the present specification, the sleep state of the subject may be one as exemplified below. That is, the sleep state may include whether the subject is sleeping, i.e., whether the subject is awake or sleeping. Also, the sleep state may include a sleep depth of the subject. Here, the sleep depth may be classified into light sleep and deep sleep, e.g., REM sleep and non-REM sleep. The sleep depth may be classified into a plurality of stages, e.g., sleep stages. Here, the sleep stages may include, for example, four stages composed of stage 1, stage 2, stage 3, and stage 4, in order from the lightest sleep. An example of the classification into the four stages will be described later with reference to FIG. 4. The sleep depth may be classified into combinations of the sleep stages and other definitions such as, for example, stage 1 and stage 2 (light sleep) and slow wave sleep (deep sleep).

Meanwhile, it is generally known that a sleep state can be classified based on, for example, a brain wave. The brain wave is classified into four waves: *β*-wave, *α*-wave, *θ*-wave, and *δ*-wave, in order from the shortest wavelength. The *β*-wave is, for example, a brain wave in a frequency of approximately between 38 Hz and 14 Hz. The *α*-wave is, for example, a brain wave in a frequency of approximately between 14 Hz and 8 Hz. The *θ*-wave is, for example, a brain wave in a frequency of approximately between 8 Hz and 4 Hz. The *δ*-wave is, for example, a brain wave in a frequency of, for example, approximately between 4 Hz and 0.5 Hz.

FIG. 4 is a graph schematically illustrating an example brain wave associated with sleep. FIG. 4 illustrates changes in the brain wave associated with sleep and predominant brain waves.

It is generally known that a person is in a sleep state when the *θ*-wave or the *δ*-wave is predominant over the *β*-wave and the *α*-wave. Here, "predominant" means that a brain wave accounts for the greatest ratio out of measured brain waves. That is, when the *θ*-wave becomes predominant over the *α*-wave (i.e., at time 0 in FIG. 4), it can be said that a person fell asleep.

As schematically illustrated in FIG. 4, it is known that a frequency of a brain wave after sleep onset (i.e., during sleep between time 0 and 7.5 in FIG. 4) periodically changes increasing and decreasing within a range of the *θ*-wave and the *δ*-wave. During sleep, the longer the brain wave, the deeper the sleep, and the shorter the brain wave, the lighter the sleep. For example, when a ratio of the *θ*-wave included in a brain wave is smaller than a predetermined value, the person is in a light sleep state (a REM sleep state). When a ratio of the *θ*-wave included in a brain wave is equal to or greater than the predetermined value, or when the δ wave is predominant, the person is in a deep sleep state (a non-REM sleep state). Accordingly, the periodic change in the frequency of the brain wave illustrated in FIG. 4 demonstrates that light sleep and deep sleep (or REM sleep and non-REM sleep) are alternately iterated.

On the other hand, it is known that, when a person awakens, a predominant brain wave changes from one with a long wavelength to another with a short wavelength. Thus, a different brainwave becomes predominant when a person is awake (i.e., at time 7.5 and later in FIG. 4), as schematically illustrated in FIG. 4.

Non-REM sleep exemplified as an index of the sleep depth described above is classified into four stages: stage 1, stage 2, stage 3 and stage 4 in order from the lightest sleep, as illustrated in FIG. 4.

It is known that a sleep state and metabolism are corelated with each other. That is, a metabolic state of the subject varies in relation to a change in the sleep state described above. Thus, in addition to the classification of the brain waves, information regarding metabolism of the subject (hereinafter, referred to as metabolism information) also enables estimation of a sleep state. Here, the metabolism information includes information regarding, for example, blood flow, breathing, sweating, body temperature, body movement, and the like of the subject when the subject awakens and sleeps and may be any metabolism information that behaves differently between at the time of awakening and sleeping or changes according to the sleep depth.

The controller 122 estimates the sleep state of the subject, based on metabolism information by utilizing the relationship between the metabolism information and a sleep state of a person. For example, in order to estimate a sleep state using the blood flow volume as one metabolism information, the controller 122 estimates a sleep state by utilizing a relationship between a change in the blood flow volume and a transition of the sleep state. The relationship between a change in the blood flow and a transition of a sleep state will be described in detail below.

First, the human brain promotes the parasympathetic nerves at the time of sleep onset. The parasympathetic nerves control a metabolic reaction of the human body. Thus, a blood flow volume at the time of sleep onset is changed in accordance with the promotion of the parasympathetic nerves. During sleep, further, the metabolic function of the human body is controlled by a behavior of the brain. Accordingly, the blood flow volume is also changed in accordance with a change in the sleep state described above, i.e., a change in an activity of the brain function. Because of such characteristics of the human body, the blood flow volume successively changes based on the sleep state, in such a manner as to increase at the time of sleep onset, to decrease as the person sleeps deeper, and then to increase as the person sleeps lighter. The controller 122 of the present embodiment can estimate a sleep state, based on a change in the blood flow volume associated with sleep as described above.

The arithmetic unit 125 calculates a statistical amount of the blood flow information calculated by the controller 122. The controller 122 estimates a sleep state based on the statistical amount of the blood flow information calculated by the arithmetic unit 125. The statistical amount of the blood flow information may be, for example, a variance of the blood flow information. The variance is a statistical value of a set of data and thus can more accurately indicate a tendency in measurement data in its entirety, rather than simply analyzing the measurement data itself of the blood flow information. That is, in a case in which the measurement apparatus 110 according to the present embodiment estimates a sleep state based on the variance, the measurement apparatus 10 can improve its usability.

Incidentally, it is known that the blood flow information includes periodic components based on the metabolism of a living body other than the pulsation of the heart, such as a respiration of a subject and vasomotion which is a local vasoconstriction motion, in addition to the periodic components based on pulsation of the heart such as the pulsating blood flow wave height and the stroke volume. A sleep state of the subject may be estimated using components of the blood flow information based on the pulsation of the heart, such as the pulsating blood flow wave height and the stroke volume. In this case, however, because the pulsation, the respiration, and the vasomotion have different cycles, it cannot be said that a result always includes, out of the periodic components included in the blood flow information, the periodic components based on the metabolism of the living body other than the pulsation of the heart, such as respiration and vasomotion. On the other hand, expressing the blood flow information using its variance enables analysis of the blood flow information as information that includes blood flow cyclic components based on the metabolism of the living body other than the pulsation of the heart, such as respiration and vasomotion that have different cycles from the pulsation of the heart. That is, the measurement apparatus 110 according to the present embodiment can improve the estimation accuracy by estimating the sleep state based on the variance of the blood flow information.

The statistical amount of the blood flow information may be any variable obtained by statistically processing measurement data such as, for example, a mean value of the blood flow information.

Here, a mode in which the controller 122 according to the present embodiment estimates a sleep state based on the variance of the blood flow information will be described in detail.

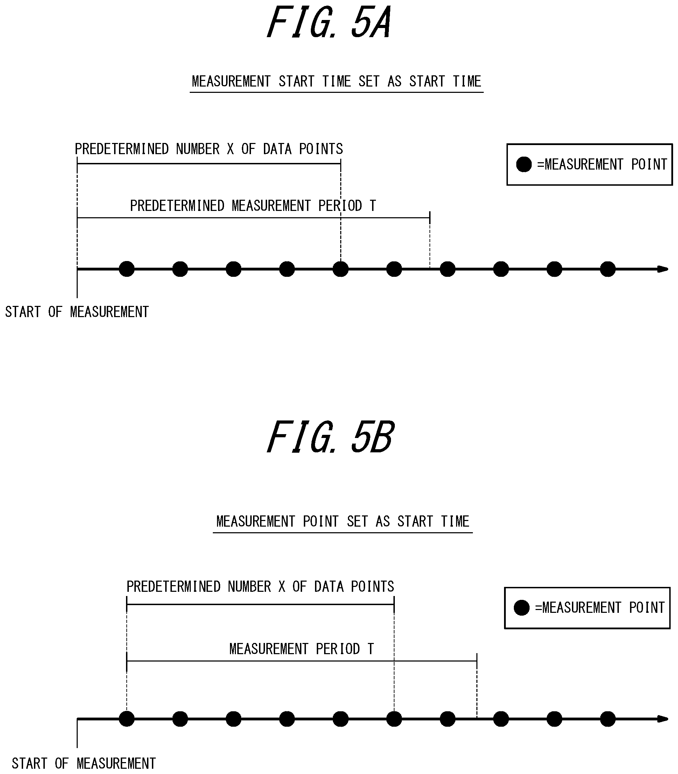

In the measurement data of the blood flow information measured by at least one of the first measurement unit 112a and the second measurement unit 112b, the arithmetic unit 125 calculates, for example, a variance of the blood flow information in each predetermined period (hereinafter, referred to as a measurement period) that is determined according to, for example, a measurement time for conducting measurement of the blood flow information, or the number of points in time (hereinafter, referred to as a measurement point) for acquiring measurement data of the blood flow information. A start time of the measurement period may be determined anytime within a range of the measurement data. FIG. 5A and 5B schematically illustrate respective methods for determining the measurement period. The start time of the measurement period may be set at, for example, a point in time at which the measurement of the blood flow information is started, as illustrated in FIG. 5A. The start time of the measurement period may be set at for example, one measurement point, as illustrated in FIG. 5B.

In a case in which the measurement period is determined based on the measurement time to conduct measurement of the blood flow information, the measurement period may be set to any predetermined period such as every 60 seconds of the measurement time, or may be set by the subject as desired. For example, in a case in which the measurement period is set to be every 60 seconds and, when data is obtained by a sensor that can obtain 50 points of the measurement data per second, 3000 points of the measurement data obtained in 60 seconds constitutes a population to be used for the calculation of the variance.

In a case in which the measurement period is determined based on the number of measurement points included in the measurement period, the measurement period may be determined based on any predetermined number of data points such as, for example, every consecutive 1000 points of the measurement data, or may be set by the subject as desired. In this case, the measurement data corresponding to the 1000 points constitutes the population to be used for the calculation of the variance. In one embodiment in which the measurement period is determined based on the number of measurement points, even when the number of measurement points constituting the population deviates from that in another embodiment in which the measurement period is determined based on the measurement time, that is, even when the measurement time (e.g., every 35 seconds of the measurement time) and sampling times of the sensor (e.g., every 1/30 seconds) differ from each other, a deviation in the number of measurement points that may occur based on their least common multiple does not need to be regarded. That is, the populations of the variances to be compared can be equalized.

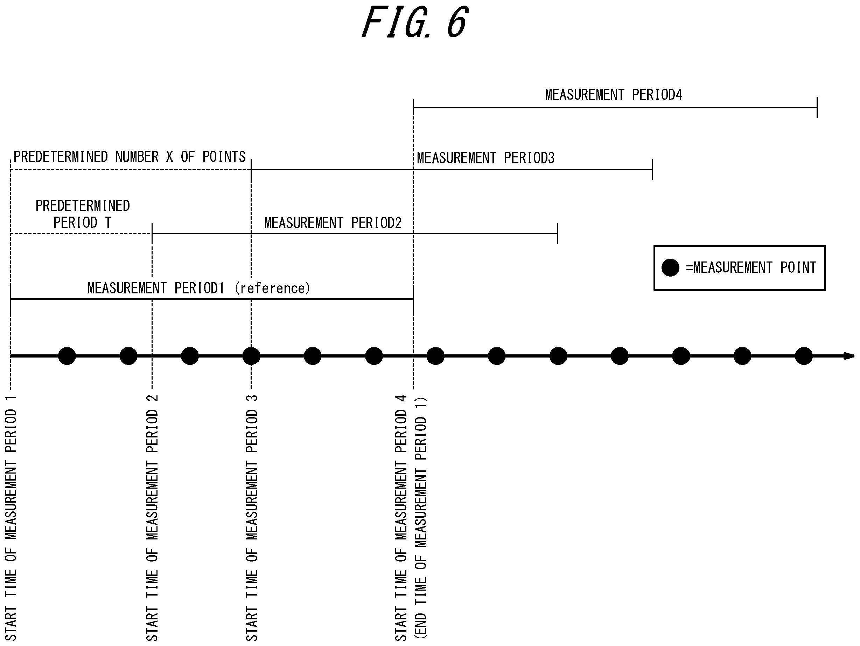

In calculating a plurality of variances of the blood flow information, the arithmetic unit 125 may set respective start times to a plurality of measurement periods in any manner. FIG. 6 illustrates an outline of a method for determining the start times of the measurement periods to successively calculate a plurality of variances. In FIG. 6, the measurement period that is used as a reference for determining a start time of a subsequent measurement period is indicated as a measurement period 1. The start time of the subsequent measurement period may be set at, for example, a point in time at which a predetermined time has elapsed from the start time of the measurement period 1 (see a measurement period 2). The start time of the subsequent measurement period may be set at, for example, a predetermined number of measurement points counted from the start time of the measurement period 1, excluding the measurement point at the start time (see a measurement period 3). In particular, for example, out of the measurement points for measuring the blood flow information included in the measurement period 1, a first measurement point excluding one at the start time of the measurement period 1 may be set as the start time of the subsequent measurement period. The start time of the subsequent measurement period may be set at, for example, end time of the measurement period 1 (see a measurement period 4). In measuring plurality of variances, the measurement periods for calculating the respective variances have the same ranges.

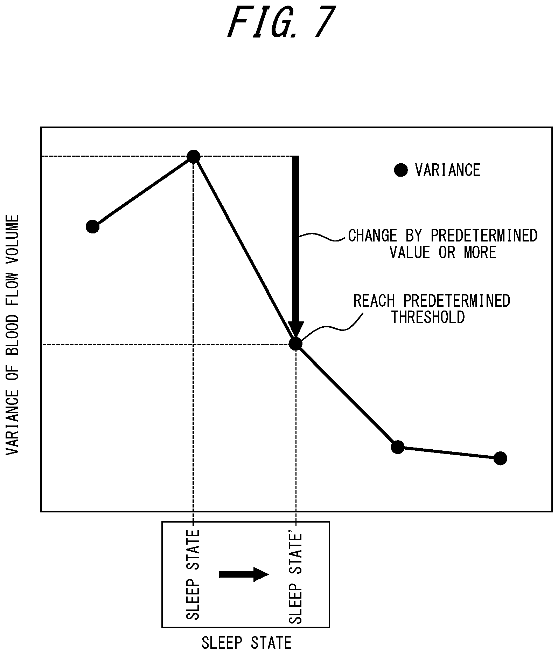

The controller 122 can determine the measurement periods as described above and successively calculate the variances. FIG. 7 illustrates a schematic diagram of a method for estimating a sleep state based on changes in the variance in a case in which the variance is successively calculated. When the controller 122 determines that a newly calculated variance has changed by a predetermined value or more with respect to a previously calculated variance or that the newly calculated variance equals a predetermined threshold, the controller 22 determines that a sleep state of the subject has changed or that the subject is in a specific sleep state and estimates the sleep state accordingly.

### Second Embodiment

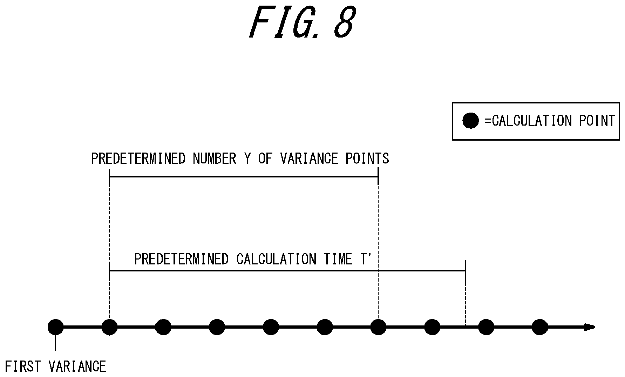

Hereinafter, a second embodiment of the electronic apparatus 100 will be described with reference to FIG. 8. Note that descriptions of features that overlap with the features of the first embodiment are omitted, and features different from the features of the first embodiment will be mainly described.

In estimating a sleep state, a representative value of a variance may be used in lieu of the variance. Although the usability of the measurement apparatus 110 can be improved by estimating a sleep state using the variance as described above, the representative value of the variances is also used in the present embodiment. As a result, the variance itself can be further standardized and smoothed, and the analysis is simplified. Thus, the measurement apparatus 110 can further improve its usability.

Hereinafter, an example method for calculating a representative value of a variance of blood flow information will be described.

In a mode in which the controller 122 estimates a sleep state of the subject based on the representative value of the variance, the arithmetic unit 125 determines a predetermined calculation period for calculating the representative value of the variance. The calculation period may be determined based on a period for calculating a variance or the number of points in time (hereinafter, referred to as a calculation point) for calculating variance data of the blood flow information included in the calculation period. FIG. 8 is a schematic diagram illustrating a method for determining the calculation period. Here, the method for determining the calculation period will be described in detail below.

A case in which the calculation period is determined based on a period corresponding to a predetermined measurement time of the blood flow information in the calculated variance data of the blood flow information will be described. Because the variance of the blood flow information is successively calculated as described above, the measurement time of the blood flow information and the calculated variance of the blood flow information have the same sampling rates. That is, the measured blood flow information and the calculated variance of the blood flow information are temporally correlated. Thus, when the variance of the blood flow information is calculated based on the measurement time as described above, the plot of the variance corresponds to continuous measurement points of the blood flow information. For example, in using a sensor that acquires 1 point of the blood flow information every 1/40 seconds, each 1 point of a variance calculated based on the acquired blood flow information serves as a plot corresponding to a measurement point of the blood flow information acquired every 1/40 seconds. Accordingly, the plot of the variance may be expressed as a plot of temporal variation. That is, the calculation period may be determined based on time as illustrated in FIG. 8, i.e., a period for calculating a variance (hereinafter, referred to as a calculation period).

In a case in which the calculation period is determined based on the number of calculation points included in the calculation period, the calculation period is determined based on the number of variance data points, as illustrated in FIG. 8.

Further, the calculation period may be determined with reference to, for example, a biological reaction of the subject. The biological reaction of the subject may be, for example, a change in a brain wave occurred while the subject is sleeping. The calculation period may be determined with reference to, for example, the metabolism information of the subject. As the metabolism information of the subject, for example, information regarding an increase and/or a decrease in the blood flow, information regarding respiratory strength, information regarding a change in respiratory cycles, information regarding a change in a sweat rate, information regarding a change in body temperature, or information regarding a frequency of body movements, while the subject is sleeping, may be used.

The controller 122 may estimate the sleep state of the subject by successively calculating the representative values of the variances and comparing a plurality of representative values. In this case, for example, a point in time at which a predetermined time has elapsed from a start time of the calculation period may be set as a start time of a subsequent calculation period. For example, a point in time at which the number of variance calculation points counted from the start time of the calculation period reaches a predetermined number of points may be set as the start time of the subsequent calculation period. For example, end time of the calculation period may be set as the start time of the subsequent calculation period.

The representative value of the variance may be, for example, a numerical value representing a statistical value of the variance. As the representative value of the variance, for example, a variance of the variance or a mean value of the variance may be used. In this case, when the controller 122 determines that a newly calculated representative value of the variance changes by a predetermined value or more with respect to the previously calculated representative value of the variance or equals a predetermined threshold, the controller 22 determines that the sleep state of the subject has changed or that the subject is in a specific sleep state and estimates the sleep state.

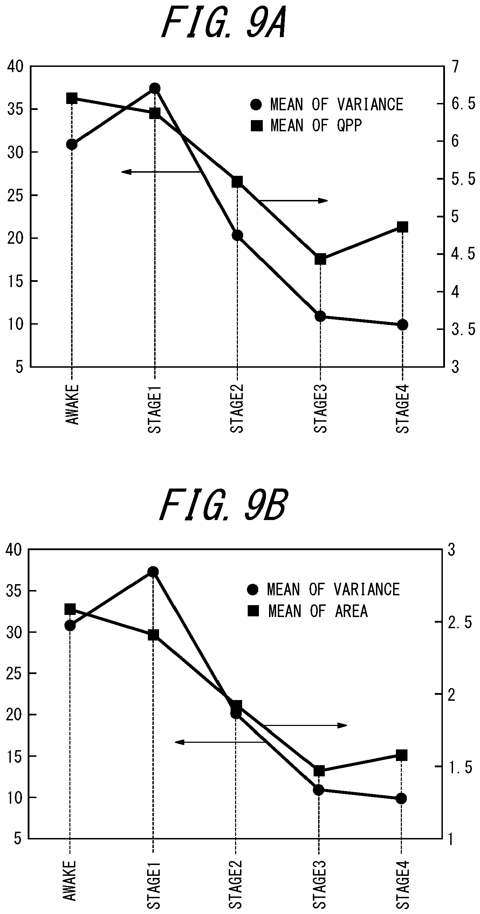

FIG. 9A and 9B are graphs on the basis of experiments in which a mean value of the variance of the blood flow and a mean value of the pulsatile blood flow wave height (QPP) or a mean value of a stroke volume (AREA) serving as examples of parameters used for estimation of the sleep state are plotted for each sleep stage. It is widely known that the blood flow volume generally tends to increase from an awake stage to sleep onset and decrease after sleep onset. As illustrated in FIGS. 9A and 9B, the plots according to the mean values of the QPP and the AREA do not necessarily follow the tendency of a change in the blood flow volume that follows a change in the sleep state (change in a sleep stage) described above. On the other hand, the plot according to a mean value of the variance of the blood flow volume exhibits a behavior that more appropriately demonstrates the tendency of the change in the blood flow volume, as compared with the plots according to the mean values of the QPP or the AREA. That is, these results support the usability of the measurement apparatus 110 demonstrated in the present specification.

The embodiment in the present specification described above is not limited to the implementation as the electronic apparatus 100. For example, the embodiment described above may be implemented as a sleep state estimation method to be executed by the electronic apparatus 100 or a program to be executed by a computer configured to control an apparatus such as the electronic apparatus 100.

Further, the function of the estimation apparatus 120 described in the above embodiment may be included as one function in another apparatus. For example, the function of the estimation apparatus 120 described in the above embodiment may be included in a mobile terminal such as a smartphone. In this case, the mobile terminal and the measurement apparatus 110 described in the above embodiment are communicatively connected to each other in, for example, a wireless manner. A photoelectric conversion signal of scattered light received by the optical detector 118 of the measurement apparatus 110 is transmitted to the mobile terminal, based on which the mobile terminal estimates a sleep state. The function of the estimation apparatus 120 described in the above embodiment may be included in an apparatus other than the mobile terminal. In this way, the function of the electronic apparatus 100 described in the above embodiment may be implemented as a system by the measurement apparatus and another apparatus.

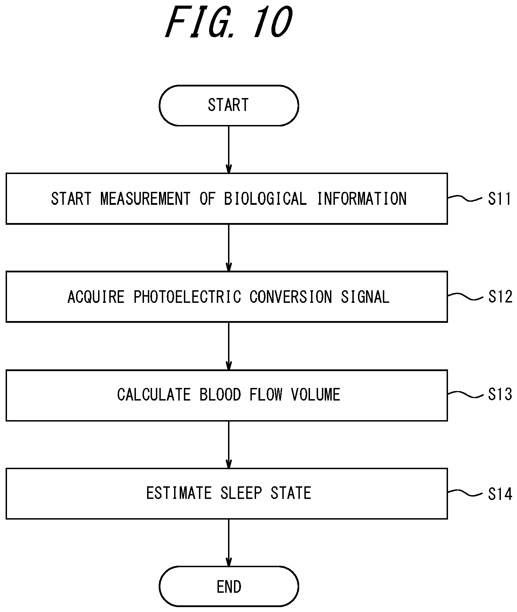

FIG. 10 is a flowchart illustrating an example of the sleep state estimation operation executed by the controller 122. The flow illustrated in FIG. 10 may start when, for example, the subject wears the measurement apparatus 110 and performs an input operation for starting the measurement using the input interface 124 of the estimation apparatus 120.

First, the controller 122 starts measuring the biological information of the subject (step S11). The controller 122 starts the measurement of the biological information by, for example, activating the sensor 116 and causing the optical emitter 117 to emit measurement light.

The controller 122 receives the photoelectric conversion signal of scattered light received by the optical detector 118 from the optical detector 118 (step S12).

The controller 122 calculates the blood flow information of the subject, based on the photoelectric conversion signal (step S13).

The controller 122 estimates the sleep state of the subject, based on the calculated blood flow information (step S14). The sleep state estimation operation may be the operation described with reference to FIGS. 9A and 9B.

The controller 122 may repeatedly execute steps S12 to S14. For example, the controller 122 may prompt the subject to wake up by outputting a sound from the speakers 119 according to the estimated sleep state.

According to the electronic apparatus 100, as described above, the sleep state of the subject can be estimated based on the blood flow information of the subject. The measurement unit can be implemented as, for example, the sensor 116 that includes the optical emitter 117 and the optical detector 118, as described in the present embodiment. The structure of the sensor 116 is smaller than, for example, known electroencephalographs and allows for a simple use thereof. Thus, the electronic apparatus 100 enables downsizing thereof and a simple estimating fashion of a sleep state, as compared with a case in which a sleep state is estimated based on a brain wave measured using an electroencephalograph. As a result, it is more convenient for the subject, and the electronic apparatus 100 improves its usability.

### REFERENCE SIGNS LIST

- 100: electronic apparatus
- 110: measurement apparatus
- 111: wearing unit
- 111a: first end portion
- 111b: second end portion
- 112a: first measurement unit
- 112b: second measurement unit
- 113a: first connecting portion
- 113b: second connecting portion
- 114a, 114b: main body
- 115a, 115b: earpiece
- 116a, 116b: sensor
- 117a, 117b: optical emitter
- 118a, 118b: optical detector
- 119a, 119b: speaker
- 120: estimation apparatus
- 1201a, 121b, 121c: communication interface
- 122: controller
- 122a: processor
- 123: memory
- 124: input interface
- 125: arithmetic unit
- 130a, 130b: cable

## Claims

1. An electronic apparatus comprising:
a measurement unit configured to measure blood flow information of a subject; and
a controller configured to estimate a sleep state of the subject based on a variance of the blood flow information.

2. The electronic apparatus according to claim 1,
further comprising an arithmetic unit configured to calculate the variance,
wherein the arithmetic unit is configured to calculate the variance, based on the blood flow information in a predetermined measurement period having a start time at any point in time, out of measured blood flow information.

3. The electronic apparatus according to claim 2,
wherein the measurement period is determined based on any one of a measurement time for measuring the blood flow information and the number of measurement points of the blood flow information.

4. The electronic apparatus according to claim 2 or 3,
wherein the arithmetic unit is configured to calculate the variance in each of a plurality of measurement periods having different start times, and
the controller is configured to estimate the sleep state based on a plurality of variances.

5. The electronic apparatus according to claim 4,
wherein the arithmetic unit is configured to successively calculate the variance by setting a start time of a measurement period subsequent to the predetermined measurement period at any one of: a point in time at which a predetermined time has elapsed from a start time of the predetermined measurement period; a point in time at which a measurement point of the blood flow information counted from the start time of the predetermined measurement period reaches a predetermined number of points; and an end time of the predetermined measurement period.

6. The electronic apparatus according to any one of claims 1 to 5,
wherein the controller is configured to estimate that the sleep state has changed or that the subject is in a specific sleep state when the variance equals a predetermined threshold.

7. The electronic apparatus according to claim 4 or 5,
wherein the controller is configured to estimate that the sleep state has changed or that the subject is in a specific sleep state when a variance calculated after a predetermined variance changes by a predetermined value or more with respect to the predetermined variance.

8. The electronic apparatus according to claim 5,
wherein the arithmetic unit is configured to calculate a representative value of the variance based on a variance in a predetermined calculation period out of successively calculated variances.

9. The electronic apparatus according to claim 8,
wherein the calculation period is determined based on any one of a variance calculation period and the number of variance calculation points.

10. The electronic apparatus according to claim 8 or 9,
wherein the arithmetic unit is configured to calculate respective representative values of variances in a plurality of calculation periods having different start times, and
the controller is configured to estimate a sleep state based on the respective representative values of the variances.

11. The electronic apparatus according to claim 10,
wherein the arithmetic unit is configured to successively calculate the representative value by setting a start time of a calculation period subsequent to a predetermined calculation period at any one of: a point in time at which a predetermined time has elapsed from a start time of the predetermined calculation period; a point in time at which a variance calculation point counted from the start time of the predetermined calculation period reaches a predetermined number of points; and an end time of the predetermined calculation period.

12. The electronic apparatus according to any one of claims 8 to 11,
wherein the controller is configured to estimate that the sleep state has changed or that the subject is in a specific sleep state when the representative value of the variance equals a predetermined threshold.

13. The electronic apparatus according to any one of claims 8 to 11,
wherein the controller is configured to determine that the sleep state has changed or that the subject is in a specific sleep state when a representative value of a variance calculated after a representative value of a predetermined variance has changed by a predetermined value or more with respect to the representative value of the predetermined variance.

14. The electronic apparatus according to any one of claims 8 to 13,
wherein respective representative values of a plurality of variances are variances or mean values obtained based on the plurality of variances.

15. The electronic apparatus according to any one of claims 1 to 14,
wherein the blood flow information includes at least one of a blood flow volume, a pulsating blood flow wave height, and a stroke volume.

16. An estimation system comprising:
a blood flow information measurement apparatus equipped with a measurement unit configured to measure blood flow information of a subject; and
an estimation apparatus equipped with a controller configured to estimate a sleep state of the subject based on a variance of the blood flow information.

17. A control method comprising:
measuring blood flow information of a subject; and
estimating a sleep state of the subject based on a variance of the blood flow information.

18. The control method according to claim 17, further comprising:
calculating the variance in each of a plurality of measurement periods having different start times; and
estimating a sleep state of the subject, based on a plurality of variances.

19. A control program for causing a computer to:
measure blood flow information of a subject; and
estimate a sleep state of the subject, based on a variance of the blood flow information.
